# FASCICULE DE BREVET EUROPEEN

(11) **EP 4 243 757 B1**
(45) Date de publication et mention de la délivrance du brevet: **05.03.2025**
(21) Numéro de dépôt: 21819926.3
(22) Date de dépôt: 10.11.2021
(51) Int. Cl.: A61H 9/00

(54) **DISPOSITIF DE MASSAGE CUTANÉ CICATRICIEL**
VORRICHTUNG ZUR MASSAGE DER NARBENHAUT
SCAR SKIN MASSAGE DEVICE

(30) Priorité: 12.11.2020 FR 2011624
(43) Date de publication de la demande: 20.09.2023
(73) Titulaire: Dreamer, 75014 Paris (FR)
(72) Inventeur: L. TIGUERT, Elenita, 75014 Paris (FR)
(74) Mandataire: IP Trust
(86) Numéro de dépôt international: PCT/FR2021/051999
(87) Numéro de publication internationale: WO 2022/101585

(56) Documents cités:
- CN-A- 105 933 501
- US-A1- 2015 141 880
- US-A1- 2017 252 261
- US-A1- 2020 188 221

## Description

### DOMAINE DE L'INVENTION

La présente invention concerne un dispositif d'assouplissement d'un tissu cutané cicatriciel post opératoire.

### ÉTAT DE LA TECHNIQUE

En France, on estime entre 49 000 et 58 000 le nombre de cancers du sein diagnostiqués chaque année et leur nombre tend à augmenter de 1,1% par an, en moyenne. Dans près de 30 à 40% des cas, les femmes (ou autres personnes atteintes) ont recours à une mastectomie, c'est-à-dire à une ablation totale ou partielle d'un ou des sein(s). Il s'agit d'une intervention chirurgicale lourde qui peut être douloureuse tant physiquement que psychologiquement.

Après une mastectomie, il est possible d'avoir recours à une reconstruction mammaire. La reconstruction mammaire fait généralement partie intégrante de la prise en charge du cancer du sein, en particulier après une chirurgie mammaire non conservatrice telle qu'une mastectomie. La reconstruction mammaire peut être immédiate, c'est à dire être réalisée en même temps que la mastectomie, ou bien différée, par le biais d'une nouvelle intervention chirurgicale quelques temps après. De manière bien connue de l'état de la technique, la reconstruction mammaire se déroule en trois temps : reconstruction du volume du sein, harmonisation des deux seins, création de la zone de l'aréole et du mamelon. Il existe actuellement trois formes différentes de reconstruction mammaire :
La reconstruction par implant : lors d'une opération chirurgicale, une prothèse mammaire est placée sous le muscle pectoral de la femme. La reconstruction par lambeau : lors d'une opération chirurgicale, des tissus musculaires (et graisseux) provenant d'une autre partie du corps sont utilisés comme greffons.

La reconstruction par injection : lors d'une opération chirurgicale, des tissus graisseux sont injectés au niveau du sein à reconstruire.

La reconstruction par implant présente des risques de complications post-opératoires importants, tels que le rejet de l'implant, le phénomène de coque péri prothétique ou diverses rétractations. Ces complications sont sources de grandes douleurs et peuvent, dans certains cas, présenter en elles-mêmes des risques infectieux. Il arrive même, dans de très rares cas, qu'un cancer du système lymphatique (Lymphome Anaplasique à Grandes Cellules associé à l'implant mammaire ou LAGC-AIM) se développe chez les patientes présentant de grandes complications post-opératoires.

La reconstruction par lambeau nécessite quant à elle sept à huit heures d'interventions chirurgicales sous anesthésie générale, réalisée en microchirurgie par des chirurgiens et chirurgiennes hautement spécialisés. Outre les risques liés à une anesthésie générale, le temps d'attente pour une telle interventions est donc long. Par ailleurs, cette reconstruction présente un risque d'échec allant de 2 à 4%, c'est-à-dire que les vaisseaux présentent un risque non négligeable de se boucher et de mener à la nécrose de la chaire greffée.

La reconstruction par injection présente peu de risques de complication post-opératoires car il s'agit d'une intervention chirurgicale relativement peu invasive, assez semblable à ce qui est pratiqué dans les traitements esthétiques non invasifs. La mastectomie laisse toutefois un tissu cicatriciel présentant une forte rigidité. Ce tissu cicatriciel ne s'étire donc pas bien et il peut donc être difficile d'injecter des tissus graisseux sous la peau rigide (donc très peu extensible) de cette région traumatisée. Cette rigidité peut ainsi aller jusqu'à empêcher de pratiquer une reconstruction par injection alors que celle-ci présente le moins de risques de complications post-opératoires et est très peu invasive.

Au vu de tous les risques, inconvénients et limitations présentés ci-dessus, seules deux femmes sur dix ont recours à une reconstruction mammaire. La présente invention a notamment pour objectif de permettre à chaque femme (ou toute autre personne) qui le souhaite d'avoir recours à une reconstruction mammaire suite à une mastectomie.

On connaît dans l'état de la technique la demande de brevet US2020188221 qui concerne un appareil de stimulation portatif comprenant :
▪ un dispositif de génération de champ de pression comprenant une unité d'entraînement et un espace creux, dans lequel un champ de pression de pressions négatives et positives alternant temporellement doit être généré dans l'espace creux par l'unité d'entraînement par un changement de volume de l'espace creux, dans lequel le creux l'espace définit une ouverture à placer sur le clitoris ;
▪ un dispositif de commande pour commander l'unité d'entraînement ;
▪ un capteur pour détecter lorsque l'appareil de stimulation est à proximité du clitoris ou lorsque l'appareil de stimulation est en contact avec le clitoris, et émettre un signal d'activation au dispositif de commande dans le cas où la proximité de l'appareil de stimulation avec le clitoris ou le contact de l'appareil de stimulation avec le clitoris est détecté ;
▪ dans lequel le dispositif de commande est destiné à activer l'unité d'entraînement lorsque le signal d'activation est présent ;
▪ le dispositif de génération de champ de pression, le dispositif de commande et le capteur étant reçus dans un boîtier.

On connaît aussi la demande de brevet US2017252261 décrivant un dispositif de massage d'un sujet vivant à traiter, comprenant un corps creux délimitant un volume interne par une paroi latérale et une paroi de liaison, la paroi latérale s'étendant entre la paroi de liaison et un bord périphérique destiné à être maintenu sur une zone de peau. du sujet à traiter ;
▪ le volume interne étant relié à un système d'aspiration afin de constituer, en maintenant le bord périphérique contre la peau, au moins une chambre d'aspiration susceptible d'être mise sous une pression négative Pi par rapport à un environnement du dispositif;
▪ le corps creux comprenant, dans son volume interne, au moins un support interne apposé à l'intérieur du corps creux et apte à comprimer la peau du sujet à traiter lorsque le volume interne est relié à une pression réduite de traitement Pi établie par le système d'aspiration ;
dans lequel le corps creux est solidaire d'un actionneur comportant une partie mobile apte à exercer sur le corps creux une force F comprimant la peau en plus de la compression qui résulte de la pression Pi.

On connaît aussi la demande de brevet US2015141880 décrivant une méthode de traitement de la douleur comprenant l'application d'une stimulation vibratoire sur la peau d'un sujet à une fréquence de 0 à 100 Hz pendant une période comprise entre 1 minute et 24 heures.

La présente invention propose ainsi une solution pour lutter contre la rigidité cicatricielle induite par la mastectomie, permettant de ce fait d'augmenter les possibilités d'avoir recours à une reconstruction mammaire par injection, moins invasive et moins dangereuse que les autres méthodes actuellement proposées.

### RÉSUMÉ

On parvient à réaliser cet objectif conformément à l'invention grâce à un dispositif de massage cutané destiné à être positionné au contact de la peau d'une personne, présentant les caractéristiques énoncées par la revendication 1.

Le dispositif comportant :
- une membrane de positionnement configurée pour
   o délimiter une zone d'action du dispositif, et
   o générer un contact étanche entre ladite zone d'action et le dispositif,
- un dispositif d'aspiration principal contrôlé par un élément de commande,
- un anneau vibreur configuré pour produire une série de vibrations selon un schéma préétabli, l'anneau vibreur étant également contrôlé par l'élément de commande, la membrane de positionnement, le disposition d'aspiration principal et l'anneau vibreur présentant chacun une symétrie de révolution autour d'un axe A, l'anneau vibreur étant fixé de manière amovible et étanche entre la membrane de positionnement et le dispositif d'aspiration principal, de manière à ce que le dispositif présente une symétrie générale de révolution autour de l'axe A. L'invention se caractérise en ce que le dispositif forme un cône de décompression qui, une fois allumé, génère, simultanément, une aspiration et une série de vibrations au niveau de la zone d'action. Ainsi, cette solution permet d'atteindre l'objectif susmentionné. En particulier, l'aspiration de la peau permet un étirement de celle-ci vers l'extérieur, créant ainsi une poche qui pourra accueillir des tissus graisseux. Le fait de combiner l'aspiration avec des vibrations permet une amélioration de la circulation sanguine et donc de redonner à la peau traumatisée une certaine souplesse et une certaine élasticité en plus de créer la poche.

Le dispositif de massage cutané selon l'invention peut comprendre une ou plusieurs des caractéristiques suivantes, prises isolément les unes des autres ou en combinaison les unes avec les autres : le dispositif comporte au moins une diode électroluminescente rouge configurée pour éclairer la zone d'action, l'élément de contrôle est une application apte à être installée sur un téléphone portable, le dispositif comporte un dispositif d'aspiration annexe, relié au dispositif d'aspiration principal de manière amovible par un conduit fluidique, chaque dispositif d'aspiration présente trois forces d'aspirations différentes, chaque dispositif d'aspiration présente une force d'aspiration basse comprise entre 90 et IOOmmHg, une force d'aspiration moyenne comprise entre 160 et 170mmHg, et une force d'aspiration haute comprise entre 240 et 250mmHg, chaque dispositif d'aspiration présente un fonctionnement discontinu, produisant une série de 40 à 60 aspirations par minute, l'anneau vibreur est configuré pour recevoir une carte mère permettant d'enregistrer le schéma préétabli de vibrations, l'anneau vibreur comporte au moins un point de vibration, chaque point de vibration générant 40 à 50 vibrations par seconde.

Est également divulgué un procédé d'étirement cutané, mis en œuvre du dispositif selon l'une quelconque des caractéristiques énumérées ci-dessus, caractérisé en ce qu'il comporte, dans l'ordre d'énonciation, les étapes suivantes : positionnement du dispositif assemblé de manière étanche selon l'axe A contre la peau d'une zone d'action d'un utilisateur, mise sous tension du dispositif, programmation et enregistrement, par l'utilisateur, dans la carte mère, au moyen de l'élément de commande, d'un schéma de vibrations, ou choix, par l'utilisateur, d'un schéma de vibrations préenregistré dans la carte mère, aspiration, par un dispositif d'aspiration, de la zone d'action, et simultanément mise en vibration, par l'anneau vibreur, de la zone d'action selon :
▪ soit un nombre de cycles définis,
▪ soit une durée indéterminée laissée à la discrétion de l'utilisateur, chaque aspiration générant un mouvement de succion de la zone d'action vers l'intérieur du dispositif de manière à étirer celle-ci, chaque vibration générant la propagation d'ondes de mouvement dans la zone d'action, de manière à la stimuler métaboliquement, le procédé permettant d'observer après plusieurs utilisations du dispositif : un gain d'élasticité et de souplesse de la zone d'action, un agrandissement de la zone d' action par étirement de celle-ci, de manière à créer une poche apte, par exemple, à recevoir une greffe ou un implant.

### DESCRIPTION DES FIGURES

L'invention sera mieux comprise, et d'autres buts, détails, caractéristiques et avantages de celle-ci apparaîtront plus clairement à la lecture de la description explicative détaillée qui va suivre, de modes de réalisation de l'invention donnés à titre d'exemples purement illustratifs et non limitatifs, en référence aux dessins annexés.
[Fig. 1] Figure 1 est une vue, éclatée, en perspective, d'un mode de réalisation de l'invention.
[Fig. 2a] Figure 2a est une vue assemblée et en perspective du mode de réalisation de la figure 1. [Fig. 2b] Figure 2b est une vue en coupe longitudinale de la vue de la figure 2a.
[Fig. 3] Figure 3 est une vue en perspective d'un anneau vibreur selon l'invention.
[Fig. 4a] Figure 4a est une vue en perspective d'un élément de commande selon le mode de réalisation de la figure 1.
[Fig. 4b] Figure 4b une vue de côté de l'élément de commande de la figure 4a.
[Fig. 4c] Figure 4c une vue de dessus de l'élément de commande de la figure 4a.
[Fig. 5] Figure 5 est un schéma fonctionnel général du dispositif selon l'invention.

### DESCRIPTION DÉTAILLÉE

Dans une première partie, sera décrit l'ensemble des pièces composant le dispositif de massage cutané 10. Son fonctionnement sera détaillé dans une deuxième partie.

### Description des pièces

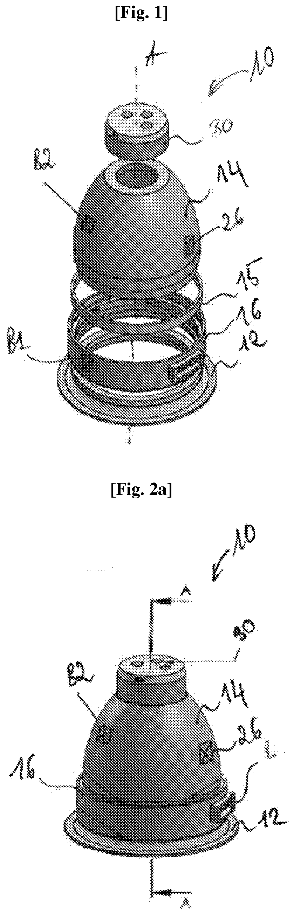

Sur la figure 1, on voit que le dispositif de massage cutané 10 selon l'invention présente trois éléments principaux destinés à s'emboiter selon un axe A : une membrane de positionnement 12, un dispositif d'aspiration principal 14, et un anneau vibreur 16.

La membrane de positionnement 12, le dispositif d'aspiration principal 14 et l'anneau vibreur 16 présentent chacun une symétrie de révolution autour de l'axe A, et lorsque ces trois éléments sont emboîtés selon l'axe A, l'anneau vibreur 16 se retrouve fixé de manière amovible et étanche entre la membrane de positionnement 12 et le dispositif d'aspiration principal 14. Ainsi, une fois assemblé, le dispositif 10 présente une symétrie générale de révolution autour de l'axe A. Dans le mode de réalisation illustré en figure 1, le dispositif 10 une fois assemblé présente une forme générale conique. Les avantages d'avoir un dispositif 10 comportant plusieurs pièces à assembler permet d'alterner les matériaux dans lesquels ces pièces sont faites. Ceci permet également de changer l'une des pièces sans changer les autres si celles-ci sont encore fonctionnelles. Par ailleurs, chaque femme (et même chaque personne de manière générale) présentant des morphologies différentes et personnelles, le fait d'être le résultat d'un assemblage de plusieurs pièces permet au dispositif 10 d'être modulable et adaptable à chacune de ces morphologies. Les différentes pièces présentent ainsi une variété de dimensions basées sur les dimensions usuelles des tailles de bonnets des articles de lingerie. Plus particulièrement, la membrane 12 est ainsi détachable du dispositif d'aspiration principal 14, ce qui permet notamment de la changer régulièrement, par exemple entre deux utilisatrices ou entre deux utilisations par la même utilisatrice, permettant une bonne désinfection.

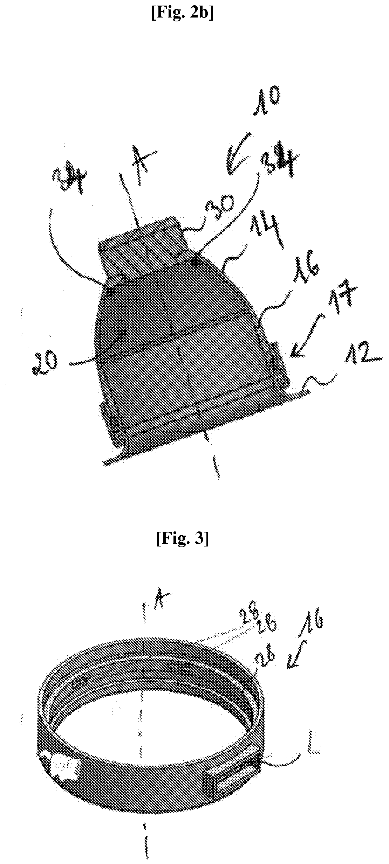

L'étanchéité entre les différents éléments à emboîter est assurée, par exemple comme illustré en figure 1, au moyen de joints annulaires 15 venant s'intercaler entre les pièces à emboîter. Cette étanchéité peut également être assurée par des mécanismes d'encliquetage annulaires 17 tels qu'illustrés en figure 2b, entre l'anneau vibreur 16 et le dispositif d'aspiration principal 14.

Le joint annulaire 15 permet également d'amortir les vibrations de l'anneau vibreur 16 au niveau du dispositif d'aspiration 14. Le joint annulaire permet également d'atténuer le bruit de l'anneau vibreur 16.

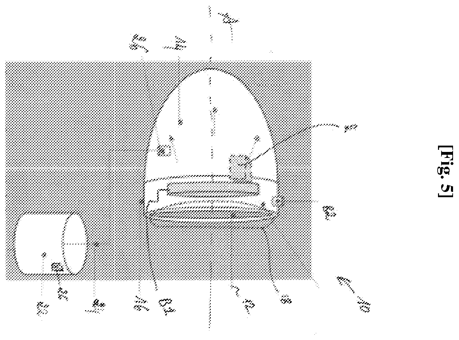

Le dispositif 10 peut être mis en marche au moyen soit : d'une batterie rechargeable B 1 (voir figures 1 et 2a), d'un moyen de branchement au secteur B2 (voir figure 5).

La batterie rechargeable B1 (illustrée en figure 5) est préférentiellement une batterie au lithium pouvant être rechargeable au moyen d'une interface USB. La batterie B 1 peut être amovible. La batterie B1 une fois rechargée permet de faire fonctionner le dispositif 10 entre 2 et 5 heures. Il est aussi possible d'utiliser le dispositif 10 en le branchant sur le secteur, par exemple lorsque l'on ne se déplace pas pendant une longue période. Le dispositif 10 peut comporter uniquement une batterie B1, uniquement des moyens de connexion au secteur B2 ou les deux.

La membrane de positionnement 12 est configurée pour
▪ délimiter une zone d'action 18 du dispositif 10 sur la peau de l'utilisatrice, la zone d'action 18 ayant été choisie avec soin, et
▪ générer et assurer un contact étanche entre ladite zone d'action 18 et le dispositif 10 pendant toute la durée d'utilisation du dispositif 10.

H est important de pouvoir délimiter avec précision une zone d'action 18 du dispositif 10 sur la peau d'une utilisatrice car il faut que l'action du dispositif 10 soit ciblée. En effet, si l'action du dispositif 10 n'est pas ciblée, l'effet serait moindre et surtout, dans l'idée de reconstruire un sein, il est très important que celui-ci soit reconstruit à un endroit précisément décidé et choisi par l'utilisatrice. La précision de l'action du dispositif 10 est donc primordiale à la bonne réussite de la reconstruction mammaire permise par le dispositif 10. Ceci est d'autant plus primordial que le dispositif 10 a vocation à être utilisé à plusieurs reprises sur la même zone d'action 18, il est donc fondamental que cette zone d'action 18 puisse être ciblée autant de fois que nécessaire avec précision et fiabilité par n'importe quelle utilisatrice.

Il est par ailleurs indispensable, pour le bon fonctionnement du dispositif 10, d'assurer une étanchéité complète tant entre les pièces emboitées comme vu ci-dessus qu'entre le dispositif 10 et la peau de l'utilisatrice.

Pour le mode de réalisation illustré aux figures 1 à 4b, la membrane de positionnement 12 présente une forme annulaire. Dans d'autres modes de réalisation, cette membrane de positionnement 12 peut présenter une forme de disque plein, comme par exemple illustré en figure 5. Cette membrane de positionnement 12 est constituée de silicone biodégradable hypoallergénique. La membrane de positionnement 12 est un élément souple pouvant se déformer. La membrane de positionnement 12 peut ainsi être aisément déformée sous l'action de l'anneau vibreur 16, par exemple. Cette souplesse lui permet également d'épouser parfaitement les courbures de l'utilisatrice et ainsi, de favoriser l'étanchéité autour de la zone d'action 18. La membrane de positionnement 12 est un élément passif. Le dispositif d'aspiration principal 14 est, quant à lui, en matériau rigide afin d'assurer une bonne prise en main et optimiser l'aspiration. Le dispositif d'aspiration principal 14 est configuré pour aspirer l'air ambiant. Il est creux, comme visible sur la figure 2b, formant ainsi une chambre de décompression 20. Cette chambre peut présenter un rayon constant ou variable et peut, d'un mode de réalisation à l'autre être très fine ou très large. A force d'aspiration constante, plus la chambre de décompression 20 est large, plus l'effet du dispositif d'aspiration principal 14 est important. Il faut toutefois que le dispositif d'aspiration principal 14 conserve une épaisseur de paroi suffisante pour supporter l'effet de l'aspiration sans se déformer. Dans le mode de réalisation illustré en figure 5, le dispositif 10 comporte en outre, un dispositif d'aspiration annexe 22, relié de manière amovible au dispositif d'aspiration principal 14 par un conduit fluidique 24.

Peu importe le nombre de dispositifs d'aspiration 14, 22, chaque dispositif d'aspiration 14, 22 présente trois forces d'aspirations différentes : une force d'aspiration basse comprise entre 90 et 100 mmHg, une force d'aspiration moyenne comprise entre 160 et 170 mmHg, et une force d'aspiration haute comprise entre 240 et 250 mmH.

En début de traitement, lors des premières utilisations du dispositif 10, lorsque la peau (le tissu cicatriciel) de la zone d'action 18 est encore très rigide et très fragile, l'utilisation d'une force d'aspiration basse permet de ménager la peau de l'utilisatrice. Plus la santé de la peau (du tissu cicatriciel) de la zone d'action 18 s'améliore, plus celle-ci présente une élasticité proche de la normale et plus la force d'aspiration peut être augmentée sans risque de provoquer des lésions.

Chaque aspiration génère une dépression dans la chambre de décompression 20. Chaque dispositif d'aspiration 14, 22 peut être mis en marche selon un fonctionnement continu ou, selon le mode de réalisation, selon un fonctionnement discontinu. Dans ce cas, chaque dispositif d'aspiration produit 14, 22 une série de 40 à 60 aspirations par minute, générant à chaque fois une dépression dans la chambre de décompression 20. Entre deux dépressions, la pression de la chambre de décompression 20 remonte. Cette remontée est soit active, c'est-à-dire que chaque dispositif d'aspiration 14, 22 peut également injecter de l'air dans la chambre de décompression 20, soit passive, c'est-à- dire que la pression de la chambre de décompression 20 revient à la pression ambiante simplement au moyen d'une valve de relâchement 26.

Cette valve de relâchement 26 peut, dans certains modes de réalisation, également servir à connecter le dispositif d'aspiration annexe 22 au dispositif d'aspiration principal 14. Dans ce cas, le dispositif d'aspiration annexe 22 présente lui-même une valve de relâchement annexe 27 jouant le même rôle.

L'anneau vibreur 16 est quant à lui, configuré pour produire une série de vibrations selon un schéma préétabli.

L'anneau vibreur 16 peut être configuré pour recevoir une carte mère permettant d'enregistrer le schéma préétabli de vibrations. Dans le mode de réalisation illustré en figures 2a et 3, l'anneau vibreur 16 est muni d'un logement L destiné à recevoir la carte mère et la batterie rechargeable B 1. Dans le mode de réalisation représenté, le logement L présente une hauteur de 8 mm et une longue de 26 mm.

De par sa forme annulaire et de par l'emboîtement étanche des différentes pièces du dispositif 10 selon l'axe A, l'anneau vibreur 16 prolonge la chambre de décompression 20 définie par le dispositif d'aspiration principal 14. Il permet ainsi de propager la dépression générée par le dispositif d'aspiration principal 14 (ou le dispositif d'aspiration annexe 22, le cas échéant) jusqu'à la membrane de positionnement 12 (ou directement jusqu'à la zone d'action 18 si la membrane 12 est annulaire). Le dispositif 10 génère ainsi une série de succions sur la zone d'action 18, c'est-à-dire sur la peau de l'utilisatrice.

On peut voir sur la figure 3 que l'anneau vibreur 16 comporte, par ailleurs, au moins un point de vibration 28, plus précisément entre trois et six points de vibration 28. Chaque point de vibration 28 peut prendre la forme d'un petit moteur électrique muni d'un balan excentrique inséré dans un creux ménagé dans une paroi interne de l'anneau vibreur 16. Chaque point de vibration génère 40 à 50 vibrations par seconde. Les points de vibration 28 fonctionnent soit simultanément ou en alternance, selon le schéma de vibration préétabli. Les mouvements ainsi générés par l'anneau vibreur 16 sont transmis, par la membrane de positionnement 12, à la zone d'action 18 et peuvent se propager au- delà. Les vibrations de l'anneau vibreur 16 génèrent des ondes qui pénètrent profondément dans la peau, leur permettant de retrouver sa souplesse et son élasticité préopératoire. Ces mouvements à action profonde permettent également d'améliorer la circulation sanguine du tissu cicatriciel. Cette stimulation de la circulation sanguine accélère l'apport en O2 dans les tissus et permet d'accélérer leur régénération.

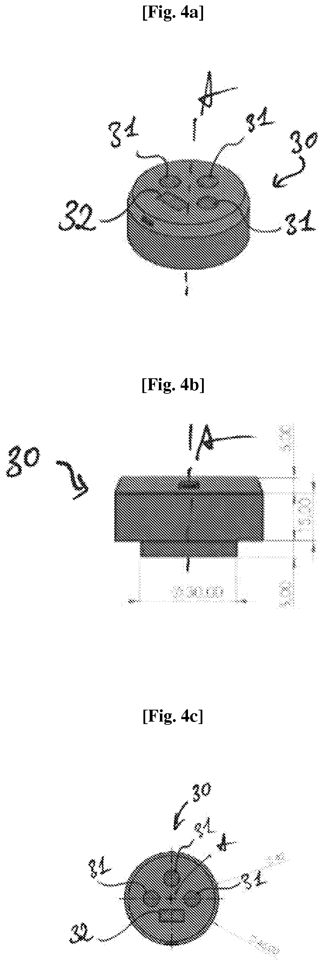

Le dispositif d'aspiration 14, chaque point de vibration 28 de l'anneau vibreur 16 ainsi que les valves de relâchement 26, 27 sont contrôlés par un élément de commande 30. Dans le mode de réalisation illustré en figures 1 et 4a, 4b et 4c, cet élément de commande 30 est positionné au somment du dispositif d'aspiration 14, formant ainsi le sommet du dispositif 10. Dans d'autres modes de réalisation, l'élément de commande 30 peut être venu de matière avec le dispositif d'aspiration 14, et l'élément de commande 30 et le dispositif d'aspiration 14 formant alors une pièce unique, plurifonctionnelle. Dans le mode de réalisation de la figure 2a, l'élément de commande 30 peut être retiré, permettant à l'utilisatrice de vérifier à l'œil nu si tout va bien ou si le dispositif 10 est bien situé, par exemple.

Dans des modes de réalisations alternatifs, l'élément de commande 30 peut être intégré à l'anneau vibreur 16. L'élément de commande 30 peut également, dans un autre mode de réalisation, prendre la forme d'une télécommande fonctionnant, par exemple, par Bluetooth. Dans certains modes de réalisation, l'élément de commande 30 peut comprendre la batterie B 1.

L'élément de contrôle 30 met en œuvre chaque schéma préétabli de vibration. Ainsi, comme visible sur les figures 2a et 5, le dispositif 10 forme un cône de décompression qui, une fois allumé, génère, simultanément, une ou plusieurs aspiration(s) et une série de vibrations au niveau de la zone d'action 18.

L'élément de contrôle 30 présente une série de boutons de commande 31 permettant : d'allumer ou d'éteindre le dispositif 10, de programmer la carte mère de l'anneau vibreur 16, ou lancer un schéma de vibration préétabli, de définir une force d'aspiration ou en changer, d'activer, en cas d'urgence, l'une des valves de relâchement 26, 27.

L'élément de contrôle 30 peut également, comme visible aux figures 4a et 4c présenter une interface de communication 32, qui permet d'indiquer les grandeurs et valeurs essentielles de l'action en cours.

Dans un mode de réalisation non illustré, l'élément de contrôle 30 est une application directement installée sur le téléphone portable de l'utilisatrice. Le dispositif 10 peut ainsi être contrôlé de manière fort pratique par le téléphone portable, ce qui rend son usage plus aisé, plus domestique et plus familier.

H faut garder à l'esprit que la peau de la zone d'action 18 est souvent très abimée, non seulement par la mastectomie mais également par les chimiothérapies ou radiothérapies subies à répétition. Ainsi, pour favoriser encore davantage la cicatrisation et la régénération de la peau de la zone d'action 18, on peut voir sur la figure 2a-b que le dispositif 10 présente au moins une diode électroluminescente (led) rouge 34 configurée pour éclairer la zone d'action 18. Plus précisément, le dispositif 10 présente, sur une paroi interne de la chambre de décompression 20, une série de petite led rouges 34. Ces petites led rouges 34 peuvent également être positionnées sur la paroi interne de l'anneau vibreur 16. Dans d'autres modes de réalisation, la série de petites led rouges 34 est située sur l'élément de commande 30, dans le cas où celui-ci vient se fixer sur le dessus du dispositif d'aspiration principal 14. Ces petites led rouges 34 émettent une lumière présentant une longueur d'onde comprise entre 620-800 nanomètres. Elles sont également contrôlées par l'élément de commande 30. Elles permettent ainsi d'éclairer, de manière contrôlée, la zone d'action 18 de manière à pouvoir pratiquer, simultanément aux vibrations et aux aspirations, de la luminothérapie. Il est recommandé d'utiliser la lumière rouge 5 à 15 minutes par jour, une à deux fois par semaine. De manière bien connue en soi, la luminothérapie utilise le spectre lumineux pour faire réagir les cellules du corps pour les rebooster et les rééquilibrer. Pour cela, elle utilise différentes couleurs qui vont intervenir en fonction des problèmes à traiter. En particulier la luminothérapie led rouge, présente des propriétés régénératrices et cicatrisantes. Elle est donc couramment utilisée pour réparer les tissus et aider à la cicatrisation. Par ailleurs, plusieurs études ont montré la capacité de la lumière rouge à soulager la douleur, et réduire le stress oxydatif. En effet, la lumière rouge stimule les mitochondries des cellules, permettant de fonctionner de façon plus efficace, accélérant ainsi leurs capacités à produire des anti-inflammatoires cicatrisants et des antioxydants protecteurs. En outre, la lumière rouge semble présenter des propriétés stimulant la production naturelle de collagène de la peau, ce qui tendrait à en augmenter l'élasticité.

Ainsi, le dispositif 10 présente-t-il une action régénératrice de la peau (par la double action de la luminothérapie et des vibrations) tout autant qu'une action préparatoire à une reconstruction mammaire (par aspiration).

### Fonctionnement

Le fonctionnement du dispositif 10 va à présent être décrit.

La mise en œuvre (ou fonctionnement) du dispositif 10 se passe, dans l'ordre d'énonciation, selon les étapes suivantes : assemblage étanche du dispositif 10 selon l'axe A, positionnement du dispositif 10 assemblé de manière étanche selon l'axe A contre la peau d'une zone d'action 18 d'une utilisatrice, mise sous tension du dispositif 10 par l'utilisatrice, programmation et enregistrement dans la carte mère, au moyen de l'élément de commande 30, d'un schéma de vibrations, ou choix d'un schéma de vibrations préenregistré dans la carte mère, aspiration, par un des deux dispositif d'aspiration 14, 22 de la zone d'action 18, et simultanément mise en vibration, par chaque point de vibration 28 l'anneau vibreur 16, selon le schéma de vibration préétabli, de la zone d'action 18 selon :
▪ soit un nombre de cycles définis,
▪ soit une durée indéterminée laissée à la discrétion de l'utilisatrice.

Chaque aspiration de l'un ou l'autre des dispositifs d'aspiration 14, 22, génère ainsi un mouvement de succion de la zone d'action 18 vers l'intérieur du dispositif 10 de manière à étirer celle-ci. Ce mouvement de succion peut être appliquée à travers la membrane de positionnement 12 si celle-ci présente une forme de disque ou appliquée directement sur la zone d'action 18 si la membrane de positionnement 12 est annulaire. Chaque vibration génère en outre la propagation d'ondes de mouvement dans la peau de la zone d'action 18, de manière à la stimuler métaboliquement, comme décrit plus haut.

Un cycle d'utilisation peut durer jusqu'à 4 heures.

Après plusieurs utilisations du dispositif 10, l'utilisatrice observe ainsi : un gain d'élasticité et de souplesse de la peau de la zone d'action 18, un agrandissement de la surface de la peau de la zone d'action 18 par étirement de celle-ci.

Cet agrandissement de la surface de la peau de la zone d'action permet de créer un espace qui, à terme, permet de recevoir une greffe de tissu graisseux. Le processus peut être répété jusqu'à obtention d'une poitrine reconstruite de la taille désirée.

Aucun rejet n'est possible puisque la greffe de tissu graisseux est issue du tissu graisseux de l'utilisatrice elle-même.

La présente invention propose ainsi une reconstruction mammaire simple, durable et non invasive. Par sa taille réduite et la présence d'une batterie rechargeable, le dispositif 10 peut être utilisé partout, à tout moment, sans gêne majeure.

### REFERENCES NUMERIQUES

10 - dispositif de massage cutané
12 - membrane de positionnement
14 - dispositif d'aspiration principal
15 - joint annulaire 16 - anneau vibreur
17 - mécanisme d'encliquetage annulaire
18 - zone d'action
20 - chambre de décompression 22 - dispositif d'aspiration annexe
24 - conduit fluidique
26 - valve de relâchement
27 - valve de relâchement annexe
28 - point de vibration 30 - élément de commande
31 - bouton de commande
32 - interface de communication
34 - diode électroluminescente rouge
B 1 - batterie rechargeable B2 - moyen de branchement au secteur

## Revendications

1. - Dispositif de massage cutané (10) pour la reconstruction mammaire destiné à être positionné au contact de la peau d'une personne, **caractérise en ce qu'**il forme un cône de décompression qui, une fois allumé, génère, simultanément, une aspiration et une série de vibrations au niveau d'une zone d'action et présente trois éléments principaux destinés à s'emboiter selon un axe A, une membrane de positionnement 12, un dispositif d'aspiration principal 14, et un anneau vibreur 16, présentant chacun une symétrie de révolution autour dudit axe A
▪ ladite membrane de positionnement (12) étant configurée pour:
▪ délimiter une zone d'action du dispositif (10), et
▪ générer un contact étanche entre une zone d'action (18) et le dispositif (10),
▪ ledit dispositif d'aspiration principal (14) étant contrôlé par un élément de commande (30),
▪ ledit anneau vibreur (16) étant configuré pour produire une série de vibrations selon un schéma préétabli, et étant également contrôlé par l'élément de commande (30),

2. - Dispositif (10) selon la revendication précédente, **caractérisé en ce que** le dispositif (10) comporte au moins une diode électroluminescente rouge (34) configurée pour éclairer la zone d'action (18).

3. - Dispositif (10) selon l'une quelconque des revendications précédentes, **caractérisé en ce que** l'élément de contrôle (30) est une application apte à être installée sur un téléphone portable.

4. - Dispositif (10) selon l'une quelconque des revendications précédentes, **caractérisé en ce que** le dispositif (10) comporte un dispositif d'aspiration annexe (22), relié au dispositif d'aspiration principal (14) de manière amovible par un conduit fluidique (24).

5. . Dispositif (10) selon l'une quelconque des revendications précédentes, **caractérisé en ce que** chaque dispositif (14, 22) d'aspiration présente trois forces d'aspirations différentes.

6. . Dispositif (10) selon la revendication précédente, **caractérisé en ce que** chaque dispositif d'aspiration (14, 22) présente une force d'aspiration basse comprise entre 90 et 100 mm Hg, une force d'aspiration moyenne comprise entre 160 et 170mm Hg, et une force d'aspiration haute comprise entre 240 et 250mmH.

7. . Dispositif (10) selon l'une quelconque des revendications précédentes, **caractérisé en ce que** chaque dispositif d'aspiration (14, 22) présente un fonctionnement discontinu, produisant une série de 40 à 60 aspirations par minute.

8. . Dispositif (10) selon l'une quelconque des revendications précédentes, **caractérisé en ce que** l'anneau vibreur (16) est configuré pour recevoir une carte mère permettant d'enregistrer le schéma préétabli de vibrations.

9. . Dispositif (10) selon l'une quelconque des revendications précédentes, **caractérisé en ce que** l'anneau vibreur (16) comporte au moins un point de vibration (28), chaque point de vibration générant 40 à 50 vibrations par seconde.

## Patentansprüche

1. Hautmassagevorrichtung (10) für die Brustrekonstruktion, die dafür bestimmt ist, mit der Haut einer Person in Kontakt gebracht zu werden, **dadurch gekennzeichnet, dass** sie einen Dekompressionskegel ausbildet, der, sobald sie eingeschaltet ist, gleichzeitig eine Saugwirkung und eine Reihe von Vibrationen an einem Wirkungsbereich erzeugt und drei Hauptelemente, die dafür bestimmt sind, sich entlang einer A-Achse einzufügen, eine Positionierungsmembran 12, eine Hauptsaugvorrichtung 14 und einen Vibrationsring 16 aufweist, die jeweils eine Rotationssymmetrie um die A-Achse aufweisen,
▪ wobei die Positionierungsmembran (12) konfiguriert ist zum:
▪ Begrenzen eines Wirkungsbereichs der Vorrichtung (10) und
▪ Erzeugen eines dichten Kontakts zwischen einem Wirkungsbereich (18) und der Vorrichtung (10),
▪ wobei die Hauptsaugvorrichtung (14) durch ein Antriebselement (30) gesteuert wird,
▪ wobei der Vibrationsring (16) konfiguriert ist, um eine Reihe von Vibrationen gemäß einem vorher festgelegten Muster zu bewirken und auch durch das Antriebselement (30) gesteuert wird,

2. Vorrichtung (10) nach dem vorstehenden Anspruch,
**dadurch gekennzeichnet, dass** die Vorrichtung (10) mindestens eine rote Leuchtdiode (34) umfasst, die konfiguriert ist, um den Wirkungsbereich (18) zu beleuchten.

3. Vorrichtung (10) nach einem der vorstehenden Ansprüche,
**dadurch gekennzeichnet, dass** das Steuerelement (30) eine Anwendung ist, die geeignet ist, auf einem Mobiltelefon installiert zu werden.

4. Vorrichtung (10) nach einem der vorstehenden Ansprüche,
**dadurch gekennzeichnet, dass** die Vorrichtung (10) eine zugehörige Saugvorrichtung (22) umfasst, die durch eine Fluidleitung (24) mit der Hauptsaugvorrichtung (14) abnehmbar verbunden ist.

5. Vorrichtung (10) nach einem der vorstehenden Ansprüche,
**dadurch gekennzeichnet, dass** jede Saugvorrichtung (14, 22) drei unterschiedliche Saugkräfte aufweist.

6. Vorrichtung (10) nach dem vorstehenden Anspruch,
**dadurch gekennzeichnet, dass** jede Saugvorrichtung (14, 22) eine niedrige Saugkraft zwischen 90 und 100 mm Hg, eine mittlere Saugkraft zwischen 160 und 170 mm Hg und eine hohe Saugkraft zwischen 240 bis 250mmHg aufweist.

7. Vorrichtung (10) nach einem der vorstehenden Ansprüche,
**dadurch gekennzeichnet, dass** jede Saugvorrichtung (14, 22) einen diskontinuierlichen Betrieb aufweist, wobei eine Reihe von 40 bis 60 Saugvorgängen pro Minute bewirkt wird.

8. Vorrichtung (10) nach einem der vorstehenden Ansprüche,
**dadurch gekennzeichnet, dass** der Vibrationsring (16) konfiguriert ist, um eine Hauptplatine aufzunehmen, die es ermöglicht, das vorher festgelegte Vibrationsmuster zu speichern.

9. Vorrichtung (10) nach einem der vorstehenden Ansprüche,
**dadurch gekennzeichnet, dass** der Vibrationsring (16) mindestens einen Vibrationspunkt (28) aufweist, wobei jeder Vibrationspunkt 40 bis 50 Vibrationen pro Sekunde erzeugt.

## Claims

1. Skin massage device (10) for breast reconstruction designed to be positioned in contact with the skin of a person, **characterized in that** said device forms a decompression cone which, when switched on, simultaneously generates suction and a series of vibrations in an action area and has three main elements designed to fit together along an axis A, a positioning membrane 12, a main suction device 14, and a vibrator ring 16, each being rotationally symmetrical about said axis A
▪ said positioning membrane (12) being configured to:
▪ delimit an action area for the device (10), and
▪ generate sealed contact between an action area (18) and the device (10),
▪ said main suction device (14) being controlled by an operating element (30),
▪ said vibrator ring (16) being configured to produce a series of vibrations according to a predetermined pattern, and also being controlled by the operating element (30),

2. Device (10) according to the preceding claim, **characterized in that** the device (10) comprises at least one red light-emitting diode (34) configured to illuminate the action area (18).

3. Device (10) according to either of the preceding claims,
**characterized in that** the control element (30) is an application suitable for installation on a cell phone.

4. Device (10) according to any of the preceding claims, **characterized in that** the device (10) comprises an auxiliary suction device (22), which is detachably connected to the main suction device (14) by a fluid channel (24).

5. Device (10) according to any of the preceding claims, **characterized in that** each suction device (14, 22) has three different suction forces.

6. Device (10) according to the preceding claim, **characterized in that** each suction device (14, 22) has a low suction force of between 90 and 100 mmHg, a medium suction force of between 160 and 170 mmHg, and a high suction force of between 240 and 250 mmHg.

7. Device (10) according to any of the preceding claims,
**characterized in that** each suction device (14, 22) has discontinuous operation, producing a series of 40 to 60 suctions per minute.

8. Device (10) according to any of the preceding claims,
**characterized in that** the vibrator ring (16) is configured to receive a motherboard for recording the predetermined vibration pattern.

9. Device (10) according to any of the preceding claims,
**characterized in that** the vibrator ring (16) comprises at least one vibration point (28), each vibration point generating 40 to 50 vibrations per second.
